(19) Europäisches Patentamt
European Patent Office
Office européen des brevets

(11) Veröffentlichungsnummer: **0 001 395**
**A2**

# (12) EUROPÄISCHE PATENTANMELDUNG

(21) Anmeldenummer: **78100777.8**

(22) Anmeldetag: **29.08.78**

(51) Int. Cl.2: **C 07 D 207/40,** C 07 D 209/52, C 07 D 209/96

(30) Priorität: **10.09.77 DE 2740848**

(43) Veröffentlichungstag der Anmeldung: **18.04.79 Patentblatt 79/8**

(84) Benannte Vertragsstaaten: **BE CH DE FR GB NL SE**

(71) Anmelder: **Bayer Aktiengesellschaft, Zentralbereich Patente,Marken und Lizenzen Bayerwerk, D-5090 Leverkusen 1 (DE)**

(72) Erfinder: **Knops, Hans-Joachim, Dr., Claudiusweg 3, D-5600 Wuppertal 1 (DE)**
Erfinder: **Draber, Wilfried, Dr., In den Birken 81, D-5600 Wuppertal 1 (DE)**
Erfinder: **Brandes, Wilhelm, Dr., Eichendorffstrasse 3, D-5653 Leich-lingen 1 (DE)**
Erfinder: **Paul, Volker, Dr., Ahornstrasse 5, D-5650 Solingen 1 (DE)**

## (54) Substituierte N-Phenyl-bernsteinsäureimide, Verfahren zu ihrer Herstellung sowie ihre Verwendung als Fungizide.

(57) Die Erfindung betrifft neue substituierte N-Phenyl-bernsteinsäureimide, ein Verfahren zu ihrer Herstellung sowie ihre Verwendung als Fungizide.

Die Verbindung der Formel

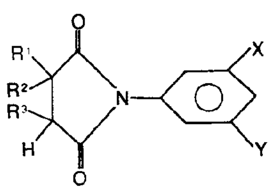

in welcher $R^1$, $R^2$, $R^3$, X und Y die in der Beschreibung angegebene Bedeutung besitzen, werden erhalten, wenn man Bernsteinsäureanhydride mit Anilinen in Gegenwart eines Verdünnungsmittels umsetzt und die entstehenden Halbamide in Gegenwart eines wasserentziehenden Mittels cyclisiert. Die erfindungsgemäßen Wirkstoffe haben ein breites Wirkungsspektrum und können angewandt werden gegen parasitäre Pilze, die oberirdische Pflanzenteile befallen oder die Pflanzen vom Boden her angreifen, sowie gegen samenübertragbare Krankheitserreger.

Als Pflanzenschutzmittel können die erfindungsgemäßen Wirkstoffe mit besonders gutem Erfolg zur Bekämpfung von Botrytis und Pellicularia, sowie als Getreidebeizmittel, insbesondere gegen Weizensteinbrand, verwendet werden.

EP 0 001 395 A2

BAYER AKTIENGESELLSCHAFT 5090 Leverkusen, Bayerwerk
Zentralbereich
Patente, Marken und Lizenzen
Slr/Th
Ia

Substituierte N-Phenyl-bernsteinsäureimide, Verfahren zu ihrer Herstellung sowie ihre Verwendung als Fungizide

Die vorliegende Erfindung betrifft neue substituierte N-Phenylbernsteinsäureimide, ein Verfahren zu ihrer Herstellung sowie ihre Verwendung als Fungizide.

Es ist bereits bekannt geworden, daß Disulfide, wie beispielsweise Tetramethyl-thiuramidisulfid, und Dithiocarbamidate, wie Zinkäthylen-1,2-bisdithiocarbamidat, gute Mittel zur Bekämpfung von pilzlichen Pflanzenkrankheiten sind (vgl. R. Wegler, Chemie der Pflanzenschutz- und Schädlingsbekämpfungsmittel, Band 2, S.59ff, Springer-Verlag 1970). Deren Wirkung ist jedoch in bestimmten Indikationsbereichen, insbesondere bei niedrigen Aufwandmengen und -konzentrationen, nicht immer voll befriedigend.

Es wurden nun als neue Verbindungen die substituierten N-Phenyl-bernsteinsäureimide der Formel

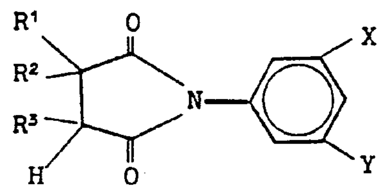

(I)

in welcher

$R^1$ für Cycloalkenyl, Isobutenyl, substituiertes Cycloalkyl, Cycloalkenyloxid, Alkenyloxid, Cycloalkanonyl, Alkanonyl oder substituiertes Alkyl steht,

$R^2$ für Wasserstoff oder Alkyl steht,

$R^3$ für Wasserstoff steht, ferner auch

$R^1$ und $R^3$ gemeinsam für eine gegebenenfalls substituierte Dimethylenbrücke stehen können, und

X und Y gleich oder verschieden sind und für Halogen stehen,

gefunden. Sie weisen starke fungizide Eigenschaften auf.

Weiterhin wurde gefunden, daß man die substituierten N-Phenylbernsteinsäureimide der Formel (I) erhält, wenn man Bernsteinsäureanhydride der Formel

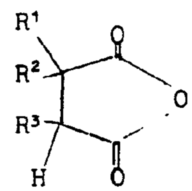

(II)

in welcher

$R^1$, $R^2$ und $R^3$ die oben angegebene Bedeutung haben,

mit Anilinen der Formel

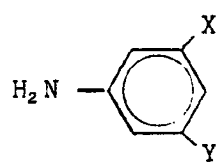

(III)

in welcher

X und Y die oben angegebene Bedeutung haben,

in Gegenwart eines Verdünnungsmittels umsetzt und die entstehenden Halbamide in Gegenwart eines wasserentziehenden Mittels cyclisiert.

In manchen Fällen kann es sich als vorteilhaft erweisen, anstelle der Bernsteinsäureanhydride der Formel (II) die entsprechenden Bernsteinsäuren einzusetzen.

Ueberraschenderweise zeigen die erfindungsgemäßen N-Phenyl-bernsteinsäureimide eine erheblich höhere fungizide Wirksamkeit, insbesondere gegen Botrytisarten, als die aus dem Stand der Technik bekannten Stoffe Tetramethyl-thiuramdisulfid und Zinkäthylen-1,2-bisdithiocarbamidat, welche bekannte Stoffe gleicher Wirkungsrichtung sind. Die erfindungsgemäßen Wirkstoffe stellen somit eine Bereicherung der Techink dar.

Verwendet man $\Delta^2$-Cyclopenten-1-yl-bernsteinsäureanhydrid und 3,5-Dichloranilin als Ausgangsstoffe, so kann der Reaktionsablauf durch das folgende Formelschema wiedergegeben werden:

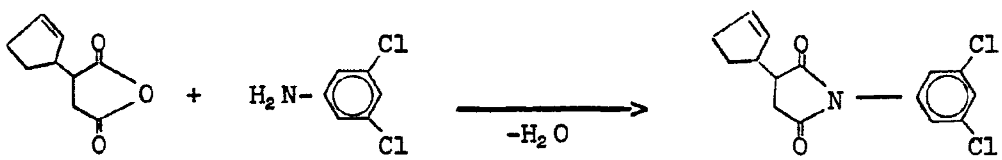


Die als Ausgangsstoffe zu verwendenden Bernsteinsäureanhydride sind durch die Formel (II) allgemein definiert. In dieser Formel steht $R^1$ vorzugsweise für Cycloalkenyl mit 4 bis 7 Kohlenstoffatomen, für Isobutenyl, für durch Halogen, Hydroxy oder Dimethylendioxo spiroförmig substituiertes Cycloalkyl mit 4 bis 7 Kohlenstoffatomen im Cycloalkylteil, für Cycloalkenyloxid und Cycloalkanonyl mit jeweils 4 bis 7 Kohlenstoffatomen, für Alkenyloxid mit 2 bis 6 Kohlenstoffatomen, für Alkanonyl mit 3 bis 7 Kohlenstoffatomen, sowie vorzugsweise für substituiertes Alkyl mit bis zu 6 Kohlenstoffatomen im Alkylteil, wobei als Substituenten vorzugsweise infrage kommen: Halogen, insbesondere Fluor, Chlor und Brom sowie Hydroxy und spiroförmig gebundenes Dimethylendioxo. $R^2$ steht vorzugsweise für Wasser-

stoff oder Alkyl mit 1 bis 4 Kohlenstoffatomen und $R^3$ steht vorzugsweise für Wasserstoff. Außerdem können $R^1$ und $R^3$ gemeinsam noch vorzugsweise mit den beiden verbindenden Kohlenstoffatomen des Fünfringes einen Spirohexyl-Rest bilden.

Die Ausgangsstoffe der Formel (II) sind bekannt (vgl. Chem. Ber. 76 (1943),S. 27 ff.))bzw. lassen sich nach den dort beschriebenen Verfahren leicht herstellen, z.B. durch Umsetzung von Maleinsäureanhydrid mit entsprechenden ungesättigten Kohlenwasserstoffen im Autoklaven, in Gegenwart eines organischen Lösungsmittels, beispielsweise Benzol, bei Temperaturen zwischen 200 und 300°C. Die Isolierung der Endprodukte erfolgt in üblicher Weise.

Die außerdem als Ausgangsstoffe zu verwendenden Aniline sind durch die Formel (III) allgemein definiert.

In dieser Formel sind X und Y gleich oder verschieden und stehen vorzugsweise für Halogen, insbesondere Fluor, Chlor oder Brom.

Die Ausgangsstoffe der Formel (III) sind allgemein bekannte Verbindungen der organischen Chemie und können auf allgemein bekannte und übliche Weise erhalten werden.

Für die erfindungsgemäße Umsetzung kommen als Verdünnungsmittel vorzugsweise inerte organische Lösungsmittel infrage. Hierzu gehören vorzugsweise aromatische Kohlenwasserstoffe, wie Benzol, Toluol oder Xylol.

Die Reaktionstemperaturen können beim erfindungsgemäßen Verfahren in einem größeren Bereich variiert werden. Im allgemeinen arbeitet man zwischen 0 und 120°C, vorzugsweise bei Raumtemperatur bis 90°C.

Die erfindungsgemäße Umsetzung wird in Gegenwart eines wasserentziehenden Mittels durchgeführt. Hierzu gehören vorzugsweise Säureanhydride, wie insbesondere Essigsäureanhydrid.

Bei der Durchführung des erfindungsgemäßen Verfahrens arbeitet man vorzugsweise in molaren Mengen. Die Isolierung der Verbindungen der Formel (I) erfolgt in üblicher Weise.

In einer besonderen Ausführungsform kann das Reaktionsgemisch auf 150 bis 200°C erhitzt werden, wobei mit Hilfe des aromatischen Lösungsmittels als Schleppmittel das Wasser abgetrennt wird. Bei Verwendung der Bernsteinsäuren als Ausgangsstoffe kann entsprechend verfahren werden.

Die erfindungsgemäßen Wirkstoffe weisen eine starke fungitoxische Wirkung auf. Sie schädigen Kulturpflanzen in den zur Bekämpfung von Pilzen notwendigen Konzentrationen nicht. Aus diesen Gründen sind sie für den Gebrauch als Pflanzenschutzmittel zur Bekämpfung von Pilzen geeignet. Fungitoxische Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die erfindungsgemäßen Wirkstoffe haben ein breites Wirkungsspektrum und können angewandt werden gegen parasitäre Pilze, die oberirdische Pflanzenteile befallen oder die Pflanzen vom

Boden her angreifen, sowie gegen samenübertragbare Krankheitserreger. Eine besonders gute Wirksamkeit entfalten sie gegen parasitäre Pilze auf oberirdischen Pflanzenteilen.

Als Pflanzenschutzmittel können die erfindungsgemäßen Wirkstoffe mit besonders gutem Erfolg zur Bekämpfung von Botrytis, Pellicularia sowie als Getreidebeizmittel, insbesondere gegen Weizensteinbrand, verwendet werden.

Als Pflanzenschutzmittel können die erfindungsgemäßen Wirkstoffe zur Saatgut-oder Bodenbehandlung und zur Behandlung oberirdischer Pflanzenteile benutzt werden.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B.

auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate; als Dispergiermittel: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungiziden,Insektiziden,Akariziden,Nematiziden,Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertigen Lösungen, Emulsionen, Suspensionen, Pulver, Pasten und Granulate angewendet werden. Die Anwendung geschieht in üblicher Weise, z.B. durch Gießen, Spritzen, Sprühen, Stäuben, Streuen, Trockenbeizen, Feuchtbeizen, Naßbeizen, Schlämmbeizen oder Inkrustieren.

Bei der Verwendung als Blattfungizide können die Wirkstoffkonzentrationen in den Anwendungsformen in einem größeren Bereich variiert werden. Sie liegen im allgemeinen zwischen 0,1 und 0,000C1 Gewichtsprozent. Vorzugsweise zwischen 0,05 und 0,0001 %.

Bei der Saatgutbehandlung werden im allgemeinen Wirkstoffmengen von 0,001 bis 50 g je Kilogramm Saatgut, vorzugsweise 0,01 bis 10 g benötigt.
Zur Bodenbehandlung sind Wirkstoffmengen von 1 bis 100 g je cbm Boden, wie insbesondere 10 bis 200 g, erforderlich.

Die erfindungsgemäßen Wirkstoffe weisen in bestimmten Konzentrationsbereichen außerdem eine bakterizide und eine wachstumsregulierende Wirkung auf.

Die vielseitigen Verwendungsmöglichkeiten gehen aus den nachfolgenden Beispielen hervor.

Beispiel A

Botrytis-Test (Bohnen) / protektiv

Lösungsmittel: 4,7 Gewichtsteile Aceton
Dispergiermittel: 0,3 Gewichtsteile Alkyl-arylpolyglykoläther
Wasser: 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man Pflanzen von Phaseolus vulgaris im 2-Blattstadium bis zur Tropfnässe. Nach 24 Stunden werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern bonitiert.

Die erhaltenen Boniturwerte werden auf Prozent Befall umgerechnet. 0 % bedeutet keinen Befall, 100 % bedeutet, daß der Befallsfleck vollständig ausgebildet ist.

Die Verbindungen entsprechend den Herstellungsbeispielen (1), (2), (5), (7) und (9) zeigen hierbei eine gute, dem bekannten Tetramethyl-thiuramidisulfid überlegene Wirkung.

**Beispiel B**

Pellicularia-Test

| | | |
|---|---|---|
| Lösungsmittel: | 11,75 Gewichtsteile | Aceton |
| Dispergiermittel: | 0,75 Gewichtsteile | Alkyl-arylpolyglykoläther |
| Wasser: | 987,50 Gewichtsteile | |
| andere Zusätze: | — Gewichtsteile | — |

Man vermischt die für die gewünschte Wirkstoffkonzentration in der Spritzflüssigkeit nötige Wirkstoffmenge mit der angegebenen Menge des Lösungsmittels und des Dispergiermittels und verdünnt das Konzentrat mit der angegebenen Menge Wasser.

Mit der Spritzflüssigkeit bespritzt man 30 etwa 2-4 Wochen alte Reispflanzen bis zur Tropfnässe. Die Pflanzen verbleiben bis zum Abtrocknen in einem Gewächshaus bei Temperaturen von 22 bis 24°C und einer relativen Luftfeuchtigkeit von etwa 70 %. Die Pflanzen werden mit einer auf Malzagar gezogenen Kultur von Pellicularia sasakii inokuliert und bei 28 bis 30°C sowie 100 % relativer Luftfeuchtigkeit aufgestellt.

Bei den mit Pellicularia sasakii infizierten Pflanzen wird der Befall nach 5 bis 8 Tagen an den Blattscheiden ebenfalls im Verhältnis zur unbehandelten, aber infizierten Kontrolle bestimmt. Die Auswertung erfolgt in Wertzahlen von 1 bis 9. 1 bedeutet 100%ige Wirkung, 3= gute Wirkung, 5 = mäßige Wirkung und 9 = keine Wirkung.

Die Verbindungen entsprechend den Herstellungsbeispielen (1), (6) und (9) zeigen eine gute, dem bekannten Zinksalz der Äthylen-bis-dithiocarbamidsäure überlegene Wirkung.

Herstellungsbeispiele

Beispiel 1

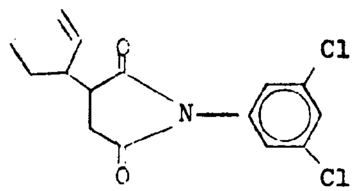

Zu 450 g (2,7 Mol) $\Delta^2$-Cyclopenten-1-yl-bernsteinsäureanhydrid in 1500 ml Benzol wird innerhalb 1 Stunde unter Rühren eine Lösung von 440 g (2,7 Mol) 3,5-Dichloranilin in 1500 ml Benzol bei Raumtemperatur zugetropft. Nach einstündigem Rühren wird der Niederschlag abgesaugt, getrocknet und zu einer Suspension von 553 g wasserfreiem Natriumacetat in 2200 ml Essigsäureanhydrid gegeben. Das Reaktionsgemisch wird 45 Minuten bei 80 bis 90°C gerührt und danach in 3000 ml Eiswasser gegossen, wo es nach kurzem Stehen auskristallisiert. Nach dem Absaugen und Trocknen erhält man 700 g (83,7 % der Theorie) N-(3,5-Dichlorphenyl)-$\Delta^2$-cyclopenten-1-yl-bernsteinsäureimid vom Schmelzpunkt 94°C.

Darstellung des Vorproduktes (vgl. Chem. Ber. 76, 48 (1943)): 1000 g Maleinsäureanhydrid und 1000 g Cyclopenten und 2000 g Benzol werden im Autoklaven während 5 Stunden bei einer Temperatur von 250°C gehalten. Es scheidet sich am Boden ein zähes Harz ab. Die überstehende Benzol-Lösung wird vom Lösungsmittel befreit und der Rückstand destilliert; Kp 12 mm Hg/160°C.

In analoger Weise werden die folgenden Verbindungen der allgemeinen Formel:

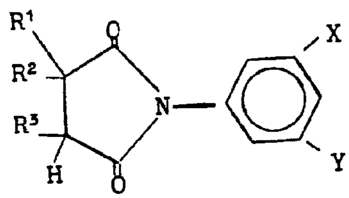

(I)

erhalten:

| Bsp. Nr. | $R^1$ | $R^3$ | $R^2$ | X | Y | Schmelzpunkt(°C) |
|---|---|---|---|---|---|---|
| 2 | 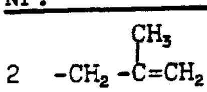 | H | H | Cl | Cl | 102-03 |
| 3 | 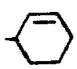 | H | H | Cl | Cl | 112-13 |
| 4 | 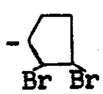 | H | H | Cl | Cl | Oel |
| 5 | 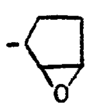 | H | H | Cl | Cl | 105-09 |
| 6 | 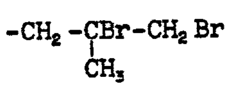 | H | H | Cl | Cl | 96-99 |
| 7 | 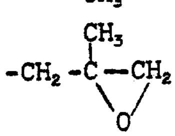 | H | H | Cl | Cl | 90-92 |

| Bsp. Nr. | $R^1$ | $R^3$ | $R^2$ | X | Y | Schmelzpunkt ($^oC$) |
|---|---|---|---|---|---|---|
| 8 | 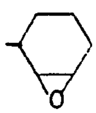 | H | H | Cl | Cl | 102-05 |
| 9 | 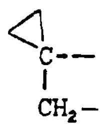 | | $CH_3$ | Cl | Cl | 124 |
| 10 | 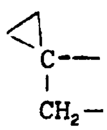 | | H | Cl | Cl | 105-10 |
| 11 | $CH_2$-CO-$CH_3$ | H | H | Cl | Cl | Glasur<br>NMR ($CCl_4$/TMS):<br>$CH_3$: δ 2,2 ppm. (sing.)<br>Arom.: δ 7,3-7,4 ppm (mutipl.)<br>IR :<br>CO : 1775 $cm^{-1}$<br>1710 $cm^{-1}$ |
| 12 | 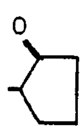 | H | H | Cl | Cl | Glasur<br>IR :<br>CO : 1780 $cm^{-1}$<br>1715 $cm^{-1}$ |
| 13 | 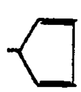 | H | H | Cl | Cl | 250 (Zers.) |

| Bsp. Nr. | $R^1$ | $R^3$ | $R^2$ | X | Y | Schmelzpunkt (°C) |
|---|---|---|---|---|---|---|
| 14 | $C(CH_3)_2-CH=O$ | H | H | Cl | Cl | 128 - 131 |
| 15 | $C(CH_3)_2$- 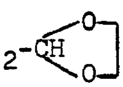 | H | H | Cl | Cl | 91 - 92 |

Fernerhin können in entsprechender Weise auch die nachfolgend aufgeführten Verbindungen der allgemeinen Formel I erhalten werden:

| $R^1$ | $R^3$ | $R^2$ | X | Y |
|---|---|---|---|---|
| 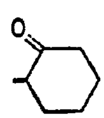 | H | H | Cl | Cl |

| $R^1$ | $R^3$ | $R^2$ | X | Y |
|---|---|---|---|---|
| 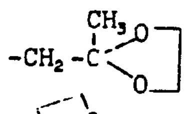 | H | H | Cl | Cl |
| 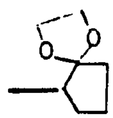 | H | H | Cl | Cl |
| 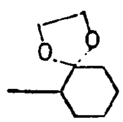 | H | H | Cl | Cl |
| 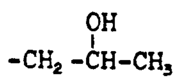 | H | H | Cl | Cl |
| 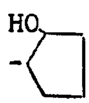 | H | H | Cl | Cl |
| 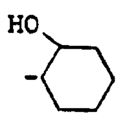 | H | H | Cl | Cl |

Patentansprüche

1) Substituierte N-Phenyl-bernsteinsäureimide der allgemeinen Formel

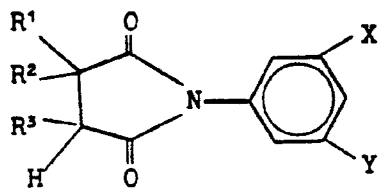

(I)

in welcher

$R^1$ für Cycloalkenyl, Isobutenyl, substituiertes Cycloalkyl, Cycloalkenyloxid, Alkenyloxid, Cycloalkanonyl, Alkanonyl oder substituiertes Alkyl steht,

$R^2$ für Wasserstoff oder Alkyl steht,

$R^3$ für Wasserstoff steht, ferner auch

$R^1$ und $R^3$ gemeinsam für eine gegebenenfalls substituierte Dimethylenbrücke stehen können, und

X und Y gleich oder verschieden sind und für Halogen stehen.

2) Verfahren zur Herstellung von substituierten N-Phenylbernsteinsäureimiden, dadurch gekennzeichnet, daß man Bernsteinsäureanhydride der Formel

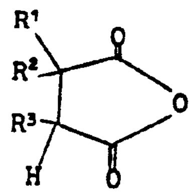

(II)

in welcher

$R^1$, $R^2$ und $R^3$ die oben angegebene Bedeutung haben,

mit Anilinen der Formel

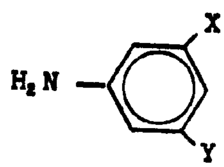

(III)

in welcher

X und Y die oben angegebene Bedeutung haben,

in Gegenwart eines Verdünnungsmittels umsetzt und die entstehenden Halbamide in Gegenwart eines wasserentziehenden Mittels cyclisiert.

3) Fungizide Mittel, gekennzeichnet durch einen Gehalt an mindestens einem substituierten N-Phenyl-bernsteinsäureimid gemäß Anspruch 1.

4) Verfahren zur Bekämpfung von Pilzen, dadurch gekennzeichnet, daß man substituierte N-Phenyl-bernsteinsäureimide gemäß Anspruch 1 auf Pilze oder ihren Lebensraum einwirken läßt.

5) Verwendung von substituierten N-Phenyl-bernsteinsäureimiden gemäß Anspruch 1 zur Bekämpfung von Pilzen.

6) Verfahren zur Herstellung von fungiziden Mitteln, dadurch gekennzeichnet, daß man substituierte N-Phenyl-bernsteinsäureimide gemäß Anspruch 1 mit Streckmitteln und/oder oberflächenaktiven Mitteln vermischt.